# EUROPÄISCHE PATENTSCHRIFT

(11) **EP 0 617 021 B1**
(45) Veröffentlichungstag und Bekanntmachung des Hinweises auf die Patenterteilung: **24.06.1998**
(21) Anmeldenummer: 94103107.2
(22) Anmeldetag: 02.03.1994
(51) Int. Cl.: C07D 213/85

(54) **Verfahren zur Herstellung von 2-Halogen-5-cyanopyridinen**
Process for the preparation of 2-halogen-5-cyanopyridines
Procédé pour la préparation de 2-halogène-5-cyanopyridines

(30) Priorität: 15.03.1993 DE 4308152
(43) Veröffentlichungstag der Anmeldung: 28.09.1994
(73) Patentinhaber: BAYER AG, 51368 Leverkusen (DE)
(72) Erfinder: Kraus, Helmut, Dr., D-51519 Odenthal (DE); Traenckner, Hans-Joachim, Dr., D-51375 Leverkusen (DE)

(56) Entgegenhaltungen:
- CHEMICAL ABSTRACTS, vol. 110, no. 21, 22. Mai 1989, Columbus, Ohio, US; abstract no. 192171r, Y. HIROSHI ET AL. 'Site selectivity in the reaction of 3-substituted pyridine 1-oxides with phosphoryl chloride' Seite 685 ;

## Beschreibung

Die Erfindung betrifft ein Verfahren zur Herstellung von 2-Halogen-5-cyano-pyridinen durch Umsetzung von Methylen-glutaconsäure-dinitrilen mit Halogenwasserstoff.

Verbindungen der obengenannten Art, beispielsweise 2-Chlor-5-cyano-pyridin, sind wichtige Zwischenprodukte für 2-Chlor-5-aminomethylpyridin (DE-OS 37 26 993) bzw, 2-Chlor-5-chlormethyl-pyridin, die ihrerseits Schlüsselverbindungen zur Herstellung von Insektiziden der Nitromethylenklasse darstellen (EP 163 855, EP 376 279, EP 425 030).

Die Chlorierung von Nicotinsäure-N-oxid-Derivaten findet bevorzugt in der 2-Stellung statt (HU 33 464; zitiert nach C.A. 103 (1985), 71 193 w), während Nicotinsäurechlorid beim Einleiten eines 15-fachen Überschusses an gasförmigem Chlor unter Belichtung 6-Chlor- nicotinsäure neben dem 2-Chlor-Derivat ergibt (JP 01/42 467 (1989); zitiert nach C.A. 111 (1989), 57 553 w). Weiterhin kann man durch bakterielle Hydroxylierung und anschließende Chlorierung zur 6-Chlor-nicotinsäure gelangen (EP 152 949, EP 72 777). Wegen der mangelhaften Raum-Zeit-Ausbeuten und der mühevollen Abtrennung von Isomeren sind jedoch beide Verfahren für eine technische Realisierung wenig geeignet.

Es war daher überraschend, daß die Cyclisierung von Methylen-glutaconsäure-dinitrilen mit Halogenwasserstoff 2-Halogen-5-cyano-pyridine in hohen Ausbeuten und frei von Isomeren ergibt.

Es wurde ein Verfahren zur Herstellung von 2-Halogen-5-cyano-pyridinen der Formel 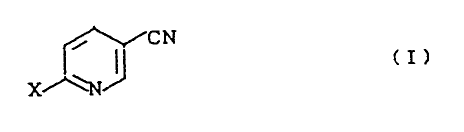 gefunden, in der
- X: für Fluor, Chlor, Brom oder Iod steht,
das dadurch gekennzeichnet ist, daß man Methylen-glutaconsäure-dinitrile der Formel

R¹-CH=C(CN)-CH=CH-CN (II),

in der
- R¹: für -OR² oder -N(R²,R³) steht, worin R² und R³ unabhängig voneinander geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ mit dem N-Atom, das sie substituieren, einen 5- bis 8-gliedrigen Ring bilden können, der ein weiteres Heteroatom aus der Gruppe von N, O und S enthalten kann,
mit Halogenwasserstoff der Formel

HX (III)

umsetzt, worin
- X: die obige Bedeutung hat.

Geradkettiges oder verzweigtes C₁-C₈-Alkyl ist beispielsweise Methyl, Ethyl, Propyl, Isopropyl, Butyl, Isobutyl, tert.-Butyl, die isomeren Pentyle, Hexyle, Octyle, bevorzugt die genannten C₁-C₄-Alkylreste.

Geradkettiges oder verzweigtes C₃-C₈-Alkenyl ist beispielsweise Allyl, die isomeren Butenyle, Pentenyle, Hexenyle oder Octenyle, bevorzugt die genannten C₃-C₄-Alkenylreste.

Geradkettiges oder verzweigtes C₂-C₈-Alkoxyalkyl ist beispielsweise Methoxymethyl, Ethoxymethyl sowie weitere Reste aus der Gruppe C₃-C₉-Alkyl, in welchem eine CH₂-Gruppe durch ein O-Atom ersetzt ist.

Geradkettiges oder verzweigtes C₄-C₈-Alkoxyalkenyl ist beispielsweise Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methyl-cyclopropyl, Dimethyl-cyclopropyl, Cyclobutyl, Methyl-cyclobutyl, Cyclopentyl, Methyl-cyclopentyl, Cyclohexyl, Methyl-cyclohexyl, Dimethyl-cyclohexyl, Cycloheptyl, Cyclocctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, das am N-Atom durch C₁-C₄-Alkyl oder durch Hydroxy-C₁-C₄-alkyl substituiert sein kann, Morpholin, Pyran, Azepin, Azocin, Isoxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können. In bevorzugter Weise seien als solche gesättigte oder ungesättigte heterocyclische Ringe Morpholin, Pyrrolidin und Piperidin, das durch C₁-C₄-Alkyl oder durch Hydroxy-C₁-C₄-alkyl substituiert sein kann, genannt.

Weiterhin können R² und R³ gemeinsam mit dem N-Atom, das sie substituieren, einen 5- bis 8-gliedrigen gesättigten oder ungesättigten Ring bilden, der ein weiteres Heteroatom aus der Gruppe N, O und S enthalten kann. Solche Ringe sind beispielsweise die oben genannten Heterocyclen.

In bevorzugter Weise werden im erfindungsgemäßen Verfahren Methylen-glutaconsäure-dinitrile eingesetzt, in denen an die Stelle von R², R³ die Substituenten R¹², R¹³ treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ gemeinsam mit dem N-Atom, das sie substituieren, einen 5- bis 8-gliedrigen Ring bilden können, der ein weiteres Heteroatom aus der Gruppe von N, O und S enthalten kann.

In besonders bevorzugter Weise werden im erfindungsgemäßen Verfahren Enamine eingesetzt, in denen an die Stelle von R¹², R¹³ die Substituenten R²², R²³ treten, die unabhängig voneinander C₁-C₄-Alkyl bedeuten, wobei weiterhin R²² und R²³ gemeinsam mit dem N-Atom, das sie substituieren, Morpholin, Pyrrolidin oder Piperidin, das durch C₁-C₄-Alkyl oder durch Hydroxy-C₁-C₄-alkyl substituiert sein kann, bedeuten.

In bevorzugter Weise tritt an die Stelle von R¹ der Substituent R¹¹ mit der Bedeutung -N(R²,R³), wobei R² und R³ unabhängig voneinander die weiter oben gegebene Bedeutung haben.

In weiterhin bevorzugter Weise tritt an die Stelle von X der Substituent X¹ mit der Bedeutung Chlor oder Brom, besonders bevorzugt an die Stelle von X¹ der Substituent Chlor.

Die erfindungsgemäße Reaktion kann beispielhaft wie folgt dargestellt werden:

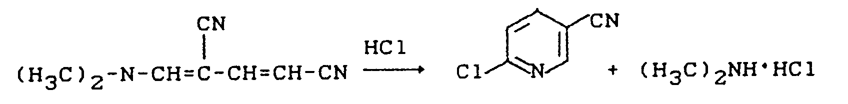

Die Umsetzung wird bei einer Temperatur von -10 bis 80°C, bevorzugt bei 20-60°C durchgeführt. Die Reaktionsdauer ist von der Temperatur und Verdünnungsmittel abhängig und beträgt 30 min bis 5 h. Der Halogenwasserstoff wird in 1 bis 40-fachem Überschuß eingesetzt, bevorzugt jedoch 1 bis 10-fach, Die Cyclisierung findet in Lösungsmitteln statt, die aus der Gruppe der Carbonsäuren, Carbonsäureamide, Alkohole, Ketone, Ether, (halogenierte) Aliphaten und Aromaten stammen. Einzelbeispiele solcher Lösungsmittel, die auch als Gemisch eingesetzt werden können, sind:

Essigsäure, Propionsäure, Dimethylacetamid, Dimethylformamid (DMF), N-Methylpyrrolidon, Ethanol, Butanol, Aceton, Methylisobutylketon, Methyl-tert.-butylether, Anisol, Tetrahydrofuran, Chloroform, Chlorethan, Dichlorethan, Petrolether, Toluol, Chlorbenzol. Für den Fall, daß in Abwesenheit von Wasser gearbeitet werden soll, kann dem Reaktionsmedium etwas Säureanhydrid, wie Acetanhydrid, zugesetzt werden.

In einer bevorzugten Ausführung verwendet man ein Gemisch aus einer protonierbaren Carbonylverbindung wie beispielsweise Essigsäure oder Dimethylformamid und einem inerten Lösungsmittel, wie beispielsweise Toluol oder Chloroform.

Der C₆-Baustein Methylen-glutaconsäure-dinitril wird in Substanz oder gelöst in einem der genannten Lösungsmittel zu einer Lösung von HF, HCl, HBr oder HJ zudosiert. Die umgekehrte Variante ist ebenso möglich.

Nach kurzer Nachreaktionszeit entfernt man überschüssiges Cyclisierungsreagens und das Lösungsmittel destillativ.

Wurde als Edukt ein Enamin gewählt, so versetzt man den Rückstand mit einer berechneten Menge Wasser, so daß man eine relativ konzentrierte wäßrige Lösung des Aminhydrohalogenids erhält. Das Produkt ist darin unlöslich und kann durch Abfiltrieren erhalten werden.

Das Enamin kann bei der Cyclisierung auch in Form der Rohproduktlösung eingesetzt werden, welche herstellungsbedingt (DE-OS 43 01 238) ein Äquivalent des entsprechenden Aminsalzes enthalten kann, wie beispielsweise eine Lösung von Dimethylaminomethylen-glutaconsäure-dinitril und Dimethylammoniumacetat in Eisessig.

Durch Überschreiten von Reaktionsparametern kann es passieren, daß entstandenes Cyanopyridin teilweise zum Carboxamid weiterreagiert. Dies ist beispielsweise mit POCl₃ wieder in das Nitril rückführbar.

### Beispiele

### Beispiel 1

150 ml 1,2-Dichlorethan wurden mit HCl-Gas gesättigt. Unter weiterem Durchleiten von HCl tropfte man bei 50°C eine 60°C heiße gesättigte Lösung von 30 g β-Dimethylaminomethylenglutaconsäure-dinitril (DIMER) in 1,2-Cl₂-ethan innerhalb von 1 h zu. Nach einer weiteren Stunde des Einleitens von HCl (schwacher Durchbruch) engte man ein und versetzte den Rückstand mit 20 ml Wasser. Nach Neutralisieren mit gesättigter NaHCO₃-Lösung wurde abgesaugt und nachgewaschen. Man erhielt 20,7 g 83,2 %iges 2-Chlor-5-cyanopyridin, entsprechend 60,9 % der theoretischen Ausbeute. Nach Umkristallisation aus Ethanol wurden Kristalle vom Schmp. 116°C erhalten. ¹H-NMR (DMSO): 7,82 (d); 8,40 (dd); 8,95 (dd).

### Beispiel 2

Analog Beispiel 1 wurden 250 ml Lösungsmittel vorgelegt und DIMER als Feststoff portionsweise zugegeben. Chlorcyanopyridin wurde in 52,9 % der theoretischen Ausbeute erhalten.

### Beispiel 3

200 ml DMF wurden bei 50°C mit HCl-Gas gesättigt. Unter weiterem Einleiten von HCl wurden innerhalb 1 h 30 g DIMER mit einer Dosierschnecke zudosiert. Nach 15 min Nachreaktionszeit engte man ein und arbeitete analog Beispiel 1 auf. Es wurden 25,3 g 87,1 %ige Ware erhalten, entsprechend 78,0 % der theoretischen Ausbeute.

### Beispiel 4

Zu einer Lösung von 22 g DMF/HCl-Addukt in 100 ml DMF tropfte man bei 30°C 13,2 g β-Dimethylamino-acrylnitril. Nach 2 h Nachreaktionszeit wurde diese Lösung unter Einleiten von HCl zu einer bei 50°C gesättigten Lösung von HCl-Gas in DMF getropft. Die Temperatur wurde durch Kühlen auf 50°C gehalten. Nach üblicher Aufarbeitung (40 ml Wasser) wurde 2-Chlor-5-cyanopyridin in 77,7 % der theoretischen Ausbeute, bezogen auf eingesetzten C₃-Baustein, erhalten.

### Beispiel 5

Analog Beispiel 3 wurde eine bei 50°C gesättigte Lösung von DIMER in DMF zugetropft. Die Ausbeute betrug 87,5 % der theoretischen Ausbeute.

### Beispiel 6

Analog Beispiel 1 legte man 180 ml 1,2-Dichlorethan und 20 ml DMF vor. 2-Chlor-5-cyanopyridin wurde in 89,4 % der theoretischen Ausbeute erhalten.

### Beispiel 7

Analog Beispiel 6 wurde bei 70°C gearbeitet. Man erhielt ein Gemisch aus 2-Chlor-5-cyanopyridin, dem Carboxamid und dem N-Formyl-carboxamid. Dieses Gemisch wurde analog Soc. 1948, 1959 mit POCl₃ umgesetzt. Nach Umkristallisation aus Petrolether wurde reines 2-Chlor-5-cyanopyridin in 72,1 % der theoretischen Ausbeute als Gesamtausbeute erhalten.

### Beispiel 8

Ein Gemisch aus 195 ml Eisessig und 5 ml Acetanhydrid wurde bei 20°C mit HCl-Gas gesättigt. Innerhalb von 1 h gab man mit Hilfe einer Dosierschnecke 30 g DIMER zu. Anschließend wurde noch 30 min lang HCl-Gas eingeleitet und der Ansatz eingeengt. Nach Verrühren mit 70 ml Wasser und Neutralisieren mit NaHCO₃-Lösung filtrierte man ab und wusch mit Wasser nach. Der Feststoff enthielt zu 41,3 % der theoretischen Ausbeute 2-Chlor-5-cyanopyridin und zu 23,8 % das Carboxamid.

### Beispiel 9

Analog Beispiel 8 wurde DIMER zu 200 ml einer 30 %igen Lösung von HBr in Eisessig dosiert. Als Hauptprodukt konnte durch GC/MS-Kopplung 2-Brom-5-cyanopyridin identifiziert werden.

### Beispiel 10

Eine Lösung von 30 g Ethoxymethylen-glutaconsäure-dinitril in 300 ml Eisessig wurde auf 0°C gekühlt und mit HCl-Gas gesättigt. Man ließ den Ansatz auf Raumtemperatur kommen und über Nacht stehen. Nach Einengen wurde mit 70 ml Wasser verrührt und mit NaHCO₃-Lösung neutralisiert. Nach Abfiltrieren und Umkristallisieren aus Ethanol erhielt man in 73,5 % der theoretischen Ausbeute 2-Chlor-5-cyanopyridin.

### Beispiel 11

Pyrrolidinomethylen-glutaconsäure-dinitril wurde analog Beispiel 5 mit HCl-Gas cyclisiert. 2-Chlor-5-cyanopyridin wurde in 83,3 % der theoretischen Ausbeute erhalten.

## Patentansprüche

1. Verfahren zur Herstellung von 2-Halogen-5-cyanopyridinen der Formel 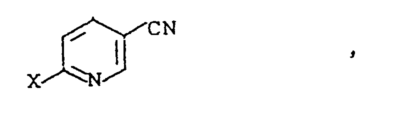 in der
X für Fluor, Chlor, Brom oder Iod steht,
dadurch gekennzeichnet, daß man Methylen-glutaconsäure-dinitrile der Formel
R¹-CH=C(CN)-CH=CH-CN ,
in der
R¹ für -OR² oder -N(R²,R³) steht, worin R² und R³ unabhängig voneinander geradkettiges oder verzweigtes C₁-C₈-Alkyl, C₃-C₈-Alkenyl, C₂-C₈-Alkoxyalkyl, C₄-C₈-Alkoxyal-kenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ mit dem N-Atom, das sie substituieren, einen 5- bis 8-gliedrigen Ring bilden können, der ein weiteres Heteroatom aus der Gruppe von N, O und S enthalten kann,
mit Halogenwasserstoff der Formel
HX
umsetzt, worin
X die obige Bedeutung hat.

2. Verfahren nach Anspruch 1, dadurch gekennzeichnet, daß an die Stelle von R² und R³ die Substituenten R¹² und R¹³ treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ gemeinsam mit dem N-Atom, das sie substituieren, einen 5- bis 8-gliedrigen Ring bilden können, der ein weiteres Heteroatom aus der Gruppe von N, O und S enthalten kann.

3. Verfahren nach Anspruch 2, dadurch gekennzeichnet, daß an die Stelle von R¹² und R¹³ die Substituenten R²², R²³ treten, die unabhängig voneinander C₁-C₄-Alkyl bedeuten, wobei weiterhin R²² und R²³ gemeinsam mit dem N-Atom, das sie substituierten, Morpholin, Pyrrolidin oder Piperidin, das durch C₁-C₄-Alkyl oder durch Hydroxy-C₁-C₄-alkyl substituiert sein kann, bedeuten.

4. Verfahren nach Anspruch 1, dadurch gekennzeichnet, daß an die Stelle von R¹ der Substituent R¹¹ mit der Bedeutung -N(R²,R³) tritt, wobei R² und R³ unabhängig voneinander die weiter oben angegebene Bedeutung haben.

5. Verfahren nach Anspruch 1, dadurch gekennzeichnet, daß die Umsetzung bei einer Temperatur von -10°C bis +80°C, bevorzugt bei 20 bis 60°C durchgeführt wird.

6. Verfahren nach Anspruch 1, dadurch gekennzeichnet, daß die Umsetzung mit 1 bis 40 Mol, bevorzugt 1 bis 10 Mol Halogenwasserstoff pro Mol Methylenglutaconsäure-dinitril durchgeführt wird.

7. Verfahren nach Anspruch 1, dadurch gekennzeichnet, daß die Umsetzung in einem Lösungsmittel aus der Gruppe der Carbonsäuren, Carbonsäureamide, Alkohole, Ketone, Ether, der aliphatischen und aromatischen (Halogen)Kohlenwasserstoffe oder einem Gemisch von ihnen durchgeführt wird.

8. Verfahren nach Anspruch 1, dadurch gekennzeichnet, daß an die Stelle von X der Substituent X¹ mit der Bedeutung Chlor oder Brom tritt und daß bevorzugt an die Stelle von X¹ der Substituent Chlor tritt.

## Claims

1. Process for the preparation of 2-halogeno-5-cyano-pyridines of the formula 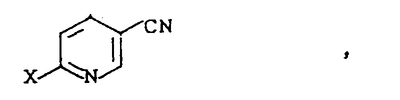 in which
X represents fluorine, chlorine, bromine or iodine,
characterized in that methylene-glutaconic acid dinitriles of the formula
R¹-CH=C(CN)-CH=CH-CN ,
in which
R¹ represents -OR² or -N(R²,R³),
in which R² and R³, independently of one another, represent straight-chain or branched C₁-C₈-alkyl, C₃-C₈-alkenyl, C₂-C₈-alkoxyalkyl, C₄-C₈-alkoxyalkenyl, C₃-C₈-cycloalkyl, C₆-C₁₂-aryl, C₇-C₁₀-aralkyl or a 5- to 8-membered saturated or unsaturated heterocyclic ring containing 1 or 2 heteroatoms from the group comprising N, O and S, where R² and R³, with the N atom on which they are substituents, may also form a 5- to 8-membered ring which may contain a further heteroatom from the group comprising N, O and S,
are reacted with hydrogen halide of the formula
HX
in which
X has the above meaning.

2. Process according to Claim 1, characterized in that the place of R² and R³ is taken by the substituents R¹² and R¹³ which, independently of one another, denote straight-chain or branched C₁-C₈-alkyl, cyclopropyl, cyclopentyl, cyclohexyl, phenyl or benzyl, where R¹² and R¹³ may also, together with the N atom on which they are substituents, form a 5- to 8-membered ring which may contain a further hetero-atom from the group comprising N, O and S.

3. Process according to Claim 2, characterized in that the place of R¹² and R¹³ is taken by the substituents R²², R²³ which, independently of one another, denote C₁-C₄-alkyl, where also R²² and R²³, together with the N atom on which they are substituents, denote morpholine, pyrrolidine or piperidine which may be substituted by C₁-C₄-alkyl or by hydroxy-C₁-C₄-alkyl.

4. Process according to Claim 1, characterized in that the place of R¹ is taken by the substituent R¹¹ having the meaning -N(R²,R³), where R² and R³, independently of one another, have the meaning given earlier.

5. Process according to Claim 1, characterized in that the reaction is carried out at a temperature of from -10°C to +80°C, preferably at from 20 to 60°C.

6. Process according to Claim 1, characterized in that the reaction is carried out with from 1 to 40 mol, preferably from 1 to 10 mol, of hydrogen halide per mole of methylene-glutaconic acid dinitrile.

7. Process according to Claim 1, characterized in that the reaction is carried out in a solvent from the group comprising carboxylic acids, carboxamides, alcohols, ketones, ethers, aliphatic and aromatic (halogeno)hydrocarbons or a mixture thereof.

8. Process according to Claim 1, characterized in that the place of X is taken by the substituent X¹ having the meaning chlorine or bromine and in that, preferably, the place of X¹ is taken by the substituent chlorine.

## Revendications

1. Procédé de préparation des 2-halogéno-5-cyanopyridines de formule 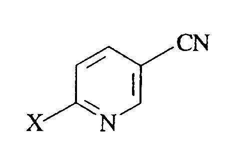 dans laquelle
X représente le fluor, le chlore, le brome ou l'iode,
caractérisé en ce que l'on fait réagir des dinitriles méthylène-glutaconiques de formule
R¹-CH=C(CN)-CH=CH-CN
dans laquelle
R¹ représente -OR² ou -N(R²,R³),
R² et R³ représentant chacun, indépendamment l'un de l'autre, un groupe alkyle en C₁-C₈, alcényle en C₃-C₈, alcoxyalkyle en C₂-C₈, alcoxyalcényle en C₄-C₈, à chaîne droite ou ramifiée, un groupe cycloalkyle en C₃-C₈, aryle en C₆-C₁₂, aralkyle en C₇-C₁₀, ou un hétérocycle saturé ou insaturé de 5 à 8 chaînons contenant un ou deux hétéroatomes choisis parmi N, O et S, R² et R³ pouvant également former ensemble et avec l'atome d'azote dont ils sont substituants un cycle de 5 à 8 chaînons qui peut contenir un autre hétéroatome choisi parmi N, O et S,
avec un halogénure d'hydrogène de formule
HX
dans laquelle
X a les significations indiquées ci-dessus.

2. Procédé selon revendication 1, caractérisé en ce que, à la place de R² et R³, il y a des substituants R¹² et R¹³ qui consistent chacun, indépendamment l'un de l'autre, en un groupe alkyle à chaîne droite ou ramifiée en C₁-C₈, cyclopropyle, cyclopentyle, cyclohexyle, phényle ou benzyle, R¹² et R¹³ pouvant également former ensemble et avec l'atome d'azote dont ils sont substituants un cycle de 5 à 8 chaînons qui peut contenir un autre hétéroatome choisi parmi N, O et S.

3. Procédé selon revendication 2, caractérisé en ce que, à la place de R¹² et R¹³, il y a des substituants R²² et R²³ qui consistent chacun, indépendamment l'un de l'autre, en un groupe alkyle en C₁-C₄, R²² et R²³ pouvant également former ensemble et avec l'atome d'azote dont ils sont substituants un cycle morpholine, pyrrolidine ou pipéridine qui peut être substitué par un groupe alkyle en C₁-C₄ ou hydroxyalkyle en C₁-C₄.

4. Procédé selon revendication 1, caractérisé en ce que, à la place de R¹, il y a un substituant R¹¹ qui consiste en -N(R²,R³), R² et R³ ayant chacun, indépendamment l'un de l'autre, les significations indiquées ci-dessus.

5. Procédé selon revendication 1, caractérisé en ce que la réaction est réalisée à des températures de -10 à +80°C, de préférence de 20 à 60°C.

6. Procédé selon revendication 1, caractérisé en ce que la réaction est réalisée avec 1 à 40 mol, de préférence 1 à 10 mol de l'halogénure d'hydrogène par mole du dinitrile méthylène-glutaconique.

7. Procédé selon revendication 1, caractérisé en ce que la réaction est exécutée dans un solvant du groupe des acides carboxyliques, des carboxamides, des alcools, des cétones, des éthers, des hydrocarbures aliphatiques et aromatiques (halogénés) ou dans un mélange de ces solvants.

8. Procédé selon revendication 1, caractérisé en ce que, à la place de X, il y a un substituant X¹ qui représente le chlore ou le brome ; et de préférence, à la place de X¹, il y a le subtituant chlore.
